# EUROPÄISCHE PATENTSCHRIFT

(11) **EP 3 582 816 B1**
(45) Veröffentlichungstag und Bekanntmachung des Hinweises auf die Patenterteilung: **30.10.2024**
(21) Anmeldenummer: 18703613.2
(22) Anmeldetag: 13.02.2018
(51) Int. Cl.: A61L 15/26, A61L 15/42, A61L 15/60, B32B 5/20, B32B 3/26, B32B 5/24, B32B 15/04, B32B 27/06, B32B 27/28, B32B 27/30, B32B 27/36, B32B 27/40, B32B 27/32, B32B 27/34

(54) **VERFAHREN ZUR HERSTELLUNG EINES KLEBSTOFFFREIEN WUNDKONTAKTVERBUNDMATERIALS**
METHOD FOR PRODUCING AN ADHESIVE-FREE WOUND CONTACT MATERIAL
PROCÉDÉ DE FABRICATION D'UN MATÉRIAU EN CONTACT AVEC UNE PLAIE EXEMPT D'ADHÉSIF

(30) Priorität: 16.02.2017 EP 17156493
(43) Veröffentlichungstag der Anmeldung: 25.12.2019
(73) Patentinhaber: Covestro Deutschland AG, 51373 Leverkusen (DE)
(72) Erfinder: WEISER, Marc-Stephan, 51515 Kürten-Dürscheid (DE); PLUG, Sascha, 51375 Leverkusen (DE); DÖRR, Sebastian, 40593 Düsseldorf (DE)
(74) Vertreter: Levpat
(86) Internationale Anmeldenummer: PCT/EP2018/053584
(87) Internationale Veröffentlichungsnummer: WO 2018/149835

(56) Entgegenhaltungen:
- EP-A1- 0 097 517
- WO-A1-2008/156285
- WO-A1-2012/150224

## Beschreibung

Die vorliegende Erfindung betrifft ein Verfahren zur Herstellung eines Verbundmaterials für Wundauflagen mit den Schritten (I) Bereitstellen einer ersten Folie, (II) Mischen eines NCO-terminierten Polyurethan-Prepolymers mit mindestens Wasser unter Erhalt einer Polyurethan-Prepolymer-Wasser-Mischung, (III) Applizieren der Polyurethan-Prepolymer-Wasser-Mischung auf mindestens einen Teil der ersten Folienoberfläche unter Bildung einer Schicht, (IV) Bedecken mindestens eines Teils der zweiten Schichtoberfläche der noch flüssigen Schicht mit einer weiteren Folie, wobei die Klebkraft zwischen der ersten Folie und der Schicht sowie zwischen der Schicht und der weiteren Folie jeweils größer ist als die Bruchspannung der Schicht, wobei das Verbundmaterial zwischen der ersten Folie und der Schicht sowie zwischen der weiteren Folie und der Schicht kein weiteres Material aufweist und wobei die erste Folie und/oder die weitere Folie ein Material ausgewählt aus der Gruppe bestehend aus einem thermoplastischen Polyurethan, einem Polyurethan oder einer Mischung hieraus beinhaltet.

Im Stand der Technik werden Verbundmaterialien für Wundauflagen beschrieben, die zwischen den verwendeten Folien und der Schaumschicht, die zur Flüssigkeitsaufnahme in der Wundauflage dient einen Klebstoff, beispielsweise in Form einer Kleberschicht, aufweisen, wobei sich das Material des Klebstoffes von dem Material der Folien bzw. der Schicht unterscheiden. Dieser Klebstoff wird benötigt, um eine ausreichende Haftung von der Schicht zu den Folien zu ermöglichen. Um diesen Klebstoff zwischen den Folien und der Schicht aufzubringen sind separate Arbeitsschritte bei der Herstellung des Verbundmaterials notwendig. Hierdurch entsteht sowohl ein zusätzlicher Zeitaufwand, Materialaufwand, Kosten sowie eine erhöhte Komplexität des Aufbaus des Verbundmaterials.

EP 0 097 517 offenbart eine Wundauflage aus einem Laminat eines verformbaren hydrophilen Schaums zwischen zwei Schichten verformbarer Netze. Die Netze sind vorzugsweise aus einem thermoplastischen Polyurethan gebildet. Bevorzugte hydrophile Schäume sind hydrophile Polyurethanschäume.

Eine Aufgabe der vorliegenden Erfindung ist es, wenigstens einen Teil der Nachteile des Standes der Technik wenigstens zu einem Teil zu verbessern.

Es ist weiterhin eine Aufgabe der Erfindung ein Verfahren zur Herstellung eines Verbundmaterials bereitzustellen, das möglichst kosten- und zeitsparend ist und möglichst wenige Verfahrensschritte aufweist.

Mindestens eine der Aufgaben wird gelöst durch ein Verfahren zur Herstellung eines Verbundmaterial gemäß des Gegenstands des Anspruch 1. Besondere Ausführungsformen sind in den abhängigen Ansprüchen beschrieben.

Gegenstand der Erfindung ist ein Verfahren zur Herstellung eines Verbundmaterials für Wundauflagen, mit den Schritten:
(I) Bereitstellen einer ersten Folie, mit einer ersten Folienoberfläche und eine zur ersten Folienoberfläche nahezu parallel verlaufenden zweiten Folienoberfläche;
(II) Mischen eines NCO-terminierten Polyurethan-Prepolymers mit mindestens Wasser unter Erhalt einer Polyurethan-Prepolymer-Wasser-Mischung,
(III) Applizieren der Polyurethan-Prepolymer-Wasser-Mischung auf mindestens einen Teil der ersten Folienoberfläche unter Bildung einer Schicht, wobei die Schicht eine erste Schichtoberfläche aufweist, die mit der ersten Folie über mindestens einen Teil der ersten Folienoberfläche in Kontakt steht und eine zweite Schichtoberfläche aufweist, die zur ersten Schichtoberfläche nahezu parallel verläuft;
(IV) Bedecken mindestens eines Teils der zweiten Schichtoberfläche der noch flüssigen Schicht mit mindestens einer weiteren Folienoberfläche einer weiteren Folie unter Erhalt des Verbundmaterials,
wobei die Klebkraft zwischen der ersten Folie und der Schicht sowie zwischen der Schicht und der weiteren Folie jeweils größer ist als die Bruchspannung der Schicht, so dass beim Versuch eine Folie von der Schicht abzuziehen, die Schicht in sich reißt, und wobei das Verbundmaterial zwischen der ersten Folie und der Schicht sowie zwischen der weiteren Folie und der Schicht kein weiteres Material aufweist und wobei die erste Folie und/oder die weitere Folie ein Material ausgewählt aus der Gruppe bestehend aus einem thermoplastischen Polyurethan, einem Polyurethan oder einer Mischung hieraus beinhaltet.

Erfindungsgemäß ist die Klebkraft dann höher als die Bruchspannung der Schicht, wenn die Schicht nicht rückstandsfrei von der ersten oder der weiteren Folie getrennt werden kann. Das bedeutet, dass beim Abtrennen der ersten oder der weiteren Folie von der Schicht, die Schicht einen Kohäsionsbruch erleidet und keinen Adhäsionsbruch bei dem die Klebkraft niedriger wäre als die Bruchspannung der Schicht. Im Gegensatz zu einem Adhäsionsbruch, der eine Trennung des Verbundes an der Haftungsoberfläche zwischen der jeweiligen Folie und der Schicht darstellen würde und ein rückstandsfreies Abtrennen der Folien von der Schicht ermöglichen würden, tritt der Kohäsionsbruch im Inneren der Schicht auf und hinterlässt Rückstände des Schichtmaterials auf der jeweiligen Folie, die abgetrennt werden soll.

In dem erfindungsgemäßen Verfahren, wird kein weiteres Material zwischen den Schritten (I) und (IV) auf die erste Folie, die Schicht oder die weitere Folie so aufgebracht, dass sich zwischen der Schicht und den Folien weiteres Material befinden würde. Das Verbundmaterial, wie es in dem erfindungsgemäßen Verfahren hergestellt wird, beinhaltet zwischen den in Schritt (I) bis (IV) bereitgestellten und verarbeiteten Materialien keine weiteren klebstoffhaltigen Materialien. Erfindungsgemäß sind die Materialien für die erste Folie sowie die weitere Folie so ausgewählt, dass die Klebkraft zwischen den Folien und der Schicht größer ist als die Bruchspannung der Schicht. Bevorzugt ist die Klebkraft in einem Bereich von 10 bis 500 %, oder bevorzugt in einem Bereich von 20 bis 450 %, oder bevorzugt in einem Bereich von 50 bis 400 %, oder bevorzugt in einem Bereich von 100 bis 300 % größer als die Bruchspannung der Schicht.

Bevorzugt weist die gebildete Schicht nach dem Applizieren und Bedecken in den Schritten (III) und (IV) eine ausreichende Klebkraft zwischen der ersten Folie und der Schicht sowie zwischen der Schicht und der weiteren Folie auf, um den Verbund auch unter Belastung zusammen zu halten.

Eine ausreichende Klebkraft bzw. eine Klebkraft zwischen den Folien und der Schicht, die größer ist als die Bruchspannung der Schicht bedeutet im Sinne der Erfindung, dass beim Versuch mindestens eine der beiden Folien, wie die erste Folie oder die weitere Folie, von der Schicht abzuziehen, mindestens auf einem Teil der ersten Folienoberfläche der ersten Folie oder der weiteren Folienoberfläche der weiteren Folie ein sichtbarer bzw. fühlbarer Rückstand an Material der Schicht verbleibt bzw. zurückbleibt. Infolgedessen kommt es bei dem Versuch mindestens eine der Folien von der Schicht abzutrennen, zu einem Kohäsionsbruch in der Schicht. Ein rückstandsfreies Abziehen der Folien von der Schicht ist bei dem Versuch mindestens eine der Folien von der Schicht abzutrennen nicht möglich. Unabhängig von der für das Auseinanderreißen des Verbundes nötigen Kraft ist die Klebkraft stets größer als die Bruchspannung des Schaumes. Die Bruchspannung ist die maximale Kraft, beispielsweise in Form von mechanischer Belastung, die das Material aushält bevor es bricht. Sie ist angegeben in Kraft pro Querschnittsfläche. Bevorzugt ist die Bruchspannung der Schicht gemessen über DIN EN ISO 527-2, mindestens 0,04 MPa.

Bevorzugt reicht die Klebkraft der Schicht zu den Folien aus, um den Verbund für eine Anwendung in einem Wundmaterial ausreichend für deren im medizinischen Umfeld bzw. im Wundversorgungsumfeld üblichen Gebrauch zusammen zu halten. Der Zusammenhalt des Verbundes bewirkt, dass ein Auseinandernehmen bzw. ein Auseinanderziehen des Verbundmaterials in seine ursprünglichen Mindestbestandteile wie die erste Folie, die Schicht und die weitere Folie nicht ohne Zerstören des Verbundmaterials, insbesondere des Schichtmaterials, möglich ist. Das hat zur Folge, dass die Mindestbestandteile nicht wie ursprünglich eingesetzt zurückerhalten werden können, ohne nicht Rückstände des Schichtmaterials auf mindestens einer der Folien aufzuweisen. So würde der Versuch die erste oder die weitere Folie von der Schicht abzuziehen dazu führen, dass die erste oder weitere Folie Teile der Schicht auf ihrer Oberfläche aufweisen. Weiterhin weist die Schicht nach Herstellung des Verbundes gemäß des erfindungsgemäßen Verfahrens bevorzugt keine Klebkraft gegenüber anderen Materialien auf, die hiermit später in Kontakt gebracht werden. Bevorzugt ist die Bruchspannung der ersten und/oder der weiteren Folie größer als die Bruchspannung der Schicht. Bevorzugt ist die Bruchspannung der ersten und/oder der weiteren Folie größer als die Klebkraft zwischen der jeweiligen Folie und der Schicht.

Auch, wenn das Verbundmaterial selbst innerhalb des Verbundes klebstofffrei aufgebaut ist, kann auf der Außenseite des Verbundes, also auf der Oberfläche der ersten Folie oder der weiteren Folie, die jeweils von der Schicht abgewandt angeordnet ist, ein weiteres Material, wie beispielsweise eine dritte Folie über eine Klebstoffschicht an den Verbund angeklebt werden.

Das Bereitstellen der ersten Folie kann auf jede Art erfolgen, die der Fachmann hierfür auswählen würde. Bevorzugt erfolgt das Bereitstellen in Schritt (I) mit Hilfe eines Rolle zu Rolle Prozesses. Es ist jedoch auch denkbar, dass zugeschnittene Folien auf einer bevorzugt flächigen Oberfläche bereitgestellt werden, die mindestens so groß ist wie die erste Folie. Bevorzugt weist die erste und/oder die weitere Folie Aussparungen, bevorzugt in Form von Löchern auf.

Das Mischen in Schritt (II) kann auf jede Art erfolgen, die der Fachmann hierfür auswählen würde. Bevorzugt erfolgt das Mischen in Schritt (II) mit Hilfe eines Zweikomponenten-Niederdruck-Mischaggregats mit dynamischem, statisch-dynamischem oder statischem Rührer, oder einem Rührer ausgewählt aus der Gruppe bestehend aus einem Magnetrührer, einem Labordissolver, einem Dispermaten oder einem anderen dem Fachmann bekannten, zum Mischen von Flüssigkeiten geeigneten Gerät. Bevorzugt wird die Polyurethan-Prepolymer-Wasser-Mischung in einer Menge von 0,1 1 bis 10000 1 in Schritt (II) hergestellt. Bevorzugt wird die Polyurethan-Prepolymer-Wasser-Mischung durch das Zusammenführen mindestens eines NCO-terminierten Polyurethan-Prepolymer und Wasser hergestellt. Das NCO-terminierte Polyurethan-Prepolymer und Wasser reagieren unter Bildung von Harnstoffgruppen und CO₂-Abspaltung, so dass ein Polyurethanschaum erhalten wird. Üblicherweise liegt das Prepolymer in flüssiger Form vor.

Bevorzugt ist das NCO-terminierte Polyurethan-Prepolymer erhältlich aus der Reaktion einer Reaktionsmischung umfassend ein Polyisocyanat und Polyol. Das Polyisocyanat weist bevorzugt eine NCO-Funktionalität in einem Bereich von >1,5 bis 6, oder bevorzugt von 1,8 bis 5, oder bevorzugt von 2 bis 4, insbesondere von 2 auf. Geeignete Polyisocyanate sind aliphatische, aromatische araliphatische oder cycloaliphatische Polyisocyanate. Beispiele solcher geeigneter Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethy-lendiisocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandiisocyanat (Nonantriisocyanat), 1,4-Phenylendiisocyanat, 2,4- und/oder 2,6-Toluylendiisocyanat, 1,5-Naphthylendiisocyanat, 2,2'-und/oder 2,4'- und/oder 4,4'-Di-phenylmethandiisocyanat, 1,3-und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysin-diisocyanate) mit C1-C8-Alkylgruppen.

Bevorzugt ist das Polyisocyanat ein aliphatisches Polyisocyanat. Bevorzugte aliphatische Diisocyanate sind Hexamethylendiisocyanat und Isophorondiisocyanat sowie deren Gemische.

Das Polyol weist bevorzugt eine OH-Funktionalität von >1,5 bis 6 oder bevorzugt von 1,8 bis 5, oder bevorzugt von 2 bis 4, insbesondere von 3 auf. Bevorzugt ist das Polyol ausgewählt aus der Gruppe bestehend aus einem Polyetherpolyol, einem Polycarbonatpolyol, einem Polyetherpolycarbonatpolyol, einem Polyesterpolyol oder einem Gemisch aus mindestens zwei hiervon. Bevorzugt ist das Polyol ein Polyetherpolyol. Bevorzugt weist das Polyol ein Polyoxyethylengruppen enthaltendes Polyol auf. Hinsichtlich der Polyole sind Copolymere aus Ethylenoxid und Propylenoxid mit einem Ethylenoxidgehalt, bezogen auf die Gesamtmenge der enthaltenen Oxyalkylengruppen, von 60 bis 85 mol-% bevorzugt.

Alternativ kann sowohl das Wasser oder eine wässrige Mischung zu dem Prepolymer oder einer Prepolymer-Mischung hinzugefügt, oder umgekehrt das Prepolymer oder die Prepolymer-Mischung zu Wasser oder der wässrigen Mischung. Es ist ebenfalls möglich, dass zwei Stoffströme (Prepolymer/Prepolymer-Mischung und Wasser/wässrige Mischung) in einem Mischelement zusammengeführt werden.

Bevorzugt wird Schritt (II) weiterhin in Gegenwart einer Base durchgeführt. Vorzugsweise weist die Base einen pK_{b}-Wert von > 6, mehr bevorzugt > 7 auf. Besonders bevorzugt ist eine COz freisetzende Base wie Hydrogencarbonat.

Das Applizieren in Schritt (III) kann auf jede Art erfolgen, die der Fachmann hierfür auswählen würde. Bevorzugt ist das Applizieren in Schritt (III) ausgewählt aus der Gruppe bestehend aus einem Gießen, einem Beschichten, wobei bevorzugt jede Art der Beschichtung geeignet ist, einem kontaktlosen Drucken, einem Siebdrucken, einem Auftragen mittels eines Pinsels, einem Sprayen oder einer Kombination aus mindestens zwei hieraus. Bevorzugt erfolgt das Applizieren mittels eines Applikators in einem Beschichtungsverfahren, bevorzugt einem Rollenbeschichtungsverfahren oder einem Rakelbeschichtungsverfahren. Das Rakel als Applikator ist bevorzugt ausgewählt aus einer Metallrakel, einer Holzrakel, einer Kunststoffrakel, einer Gummirakel oder einer Kombination aus mindestens zwei hiervon. Statt eines Rakels können auch eine oder mehrere Walzen oder Rollen als Applikator verwendet werden. Der Spalt zwischen der zu beschichtenden Folie und dem Applikator, wie der Rakelspalt oder der Spalt zwischen Walze und erster Folie weist bevorzugt eine Dicke in einem Bereich von 100 µm bis 1 cm, oder bevorzugt in einem Bereich von 250 µm bis 5 mm auf. Der Applikator, beispielsweise das Rakel oder die Rolle, wird bevorzugt in einer Geschwindigkeit in einem Bereich von 0,1 bis 30 m/min, oder bevorzugt in einem Bereich von 1 bis 20 m/min, oder bevorzugt in einem Bereich von 2 bis 10 m/min bewegt, bzw. wird bei Verwendung eines ortsfesten Applikators, wie Rakel oder Rolle die Bahn unter dem Rakel bzw. der Rolle mit einer Geschwindigkeit in den zuvor angegebenen Geschwindigkeitsbereichen bewegt. Durch die Abspaltung von CO₂ nach Mischen der Polyurethan-Prepolymer-Wasser-Mischung in Schritt (II) entsteht aus der aufgebrachten Mischung in Schritt (III) die Schicht, bevorzugt in Form eines offenzelligen Schaums. Die Polyurethan-Prepolymer-Wasser-Mischung weist bevorzugt eine Dichte in einem Bereich von 900 g/l bis 1200 g/l, oder bevorzugt in einem Bereich von 950 g/l bis 1150 g/l, oder bevorzugt in einem Bereich von 1000 bis 1100 g/l auf Bevorzugt weist die Schicht in Form eines Schaums eine mittlere Porengröße in einem Bereich von 100 bis 700 µm, bevorzugt in einem Bereich von 120 bis 400 µm oder bevorzugt in einem Bereich von 150 bis 250 µm auf. Die entstehende Schicht in Form des Schaums weist bevorzugt eine geringere Dichte auf als die Polyurethan-Prepolymer-Wasser-Mischung bei ihrer Herstellung. Bevorzugt weist die Schicht eine Dichte auf, die in einem Bereich von 3 bis 10 mal, oder bevorzugt in einem Bereich von 3,5 bis 9,5 mal, oder bevorzugt in einem Bereich von 4 bis 9 mal größer ist als die Dichte der Polyurethan-Prepolymer-Wasser-Mischung. Bevorzugt weist die Schicht eine Dichte in einem Bereich von 90 g/l bis 350 g/l, oder bevorzugt in einem Bereich von 100 g/l bis 280 g/l, oder bevorzugt in einem Bereich von 110 bis 180 g/l auf.

Das Bedecken mindestens eines Teils der zweiten Schichtoberfläche der noch flüssigen Schicht mit einer weiteren Folie in Schritt (IV) erfolgt bevorzugt durch ein Verfahren ausgewählt aus der Gruppe bestehend aus einem Ablegen, einem Kaschieren oder einer Kombination hieraus. Bevorzugt wird die weitere Folie von Rolle zu Rolle auf die noch flüssige Schicht, die in Schritt (III) gebildet wird abgelegt.

Die erste Folie und/oder die weitere Folie beinhalten ein Material ausgewählt aus der Gruppe bestehend aus einem thermoplastischen Polyurethan, einem Polyurethan oder einer Mischung hieraus.

Bevorzugt weist die erste Folie ein Material auf, das geeignet ist zu der Schicht eine Klebkraft aufzubauen, die größer ist als die Bruchdehnung der Schicht. Bevorzugt weist die erste Folie ein Material auf, das befähigt ist eine Klebkraft zwischen der Schicht und der ersten Folie auszubilden die oberhalb von 0,5 N/20 mm, oder bevorzugt oberhalb von 1 N/20 mm, oder bevorzugt oberhalb von 2 N/20 mm, oder bevorzugt in einem Bereich von 0,5 N/20 mm bis 30 N/20 mm, oder bevorzugt in einem Bereich von 1 N/20 mm bis 25 N/20 mm, oder bevorzugt in einem Bereich von 2 N/20 mm bis 15 N/20 mm.

Die erste Folie weist bevorzugt eine Gesamtoberfläche in einem Bereich von 1 cm² bis 1000m², oder bevorzugt in einem Bereich von 10 cm² bis 500m², oder bevorzugt in einem Bereich von 50 cm² bis 100m² auf, wobei mögliche Aussparungen mit zu der Berechnung oder Vermessung der Gesamtoberfläche der ersten Folie gezählt werden. Bevorzugt nehmen die Aussparungen in der Folie eine Fläche in einem Bereich von 5 bis 50 %, oder bevorzugt in einem Bereich von 10 bis 45 %, oder bevorzugt von 15 bis 40 % der Gesamtoberfläche der ersten Folie ein.

Bevorzugt sind die Mengenverhältnisse der Materialien für die weitere Folie die gleichen wie für die erste Folie angegeben. Weiterhin bevorzugt ist die Gesamtfläche der weiteren Folie in dem gleichen Bereich wie für die erste Folie angegeben. In einer bevorzugten Ausführungsform des erfindungsgemäßen Verfahrens erfolgen mindestens die Schritte (III) und (IV) zusammengenommen, bevorzugt die Schritte (II) bis (IV) zusammengenommen, in einem Zeitraum von weniger als 120 Sekunden, bevorzugt von weniger als 90 Sekunden, oder bevorzugt von weniger als 60 Sekunden oder bevorzugt von weniger als 40 Sekunden. Bevorzugt vergehen zwischen dem Anrühren der Polyurethan-Wasser-Mischung in Schritt (II) und dem Aufbringen dieser Mischung auf die erste Folie in Schritt (III) nicht mehr als 120 Sekunden, bevorzugt nicht mehr als 90 Sekunden, oder bevorzugt von nicht mehr als 60 Sekunden oder bevorzugt von nicht mehr als 40 Sekunden. Bevorzugt vergehen zwischen dem Anrühren der Polyurethan-Wasser-Mischung in Schritt (II) und dem Aufbringen der weiteren Folie in Schritt (IV) auf die in Schritt (III) auf die erste Folie aufgebrachte Polyurethan-Wasser-Mischung nicht mehr als 120 Sekunden, bevorzugt nicht mehr als 90 Sekunden, oder bevorzugt von nicht mehr als 60 Sekunden oder bevorzugt von nicht mehr als 40 Sekunden.

Im erfindungsgemäßen Verfahren weist das Verbundmaterial zwischen der ersten Folie und der Schicht sowie zwischen der weiteren Folie und der Schicht kein weiteres Material auf. Wie bereits erwähnt, kann das Verbundmaterial jedoch auf seiner Außenseite, also auf einer Oberfläche der ersten Folie oder der weiteren Folie, die nicht mit der Schicht in Verbindung steht, weitere Materialien, wie beispielsweise Klebstoffe aufweisen.

Bevorzugt wird die Polyurethan-Prepolymer-Wasser-Mischung in Schritt (III), bevorzugt in Form einer Nassschicht, mit einer Nassschicht-Dicke, die durch den Rakelspalt bestimmt wird, in einem Bereich von 50 bis 5000 µm, bevorzugt in einem Bereich von 100 bis 3000 µm, oder bevorzugt in einem Bereich von 300 bis 2000 µm appliziert. Bevorzugt wird innerhalb von 1 bis 90 Sekunden, oder bevorzugt von 2 bis 80 Sekunden, oder bevorzugt innerhalb von 5 bis 60 Sekunden die Nassschicht von der weiteren Folie bedeckt. Die Nassschicht härtet bevorzugt innerhalb von 1 bis 10 Minuten, bevorzugt innerhalb von 2 bis 8 Minuten, oder bevorzugt innerhalb von 3 bis 6 Minuten zu mindestens 50 %, oder bevorzugt zu mindestens 60 %, oder bevorzugt zu mindestens 70 % aus, bezogen auf die Gesamtflüssigkeitsmenge, die während des gesamten Aushärtevorgangs entweicht.

In einer bevorzugten Ausführungsform des erfindungsgemäßen Verfahrens weist die Schicht eine Dicke nach dem Aushärten auf, die um das 1,5 bis 30-fache, oder bevorzugt um das 2 bis 10-fache, oder bevorzugt um das 2,5 bis 5-fache größer ist als die Dicke beim Applizieren der Polyurethan-Prepolymer-Wasser-Mischung in Schritt (III). Bevorzugt weist die Schicht nach dem Aushärten eine Dicke in einem Bereich von 70 µm bis 10 cm, oder bevorzugt in einem Bereich von 100 µm bis 5 cm, oder bevorzugt in einem Bereich von 200 µm bis 10 cm, oder bevorzugt in einem Bereich von 500 µm bis 1 cm auf.

In einer bevorzugten Ausführungsform des erfindungsgemäßen Verfahrens weist das NCO-terminierte Polyurethan-Prepolymer einen Gewichtsanteil an niedermolekularen, aliphatischen Diisocyanaten mit einer Molmasse von ≥ 140 bis ≤ 278 g/mol von ≤ 1.0 Gew.-%, bezogen auf das Prepolymer auf und ist als Komponente A) erhältlich durch Umsetzung von
A1) niedermolekularen, aliphatischen Diisocyanaten einer Molmasse von ≥ 140 bis ≤ 278 g/mol mit
A2) di- bis hexafunktionellen Polyalkylenoxiden einer OH-Zahl von ≥ 22.5 bis ≤ 112 mg KOH/g und einem Ethylenoxidanteil von ≥ 50 bis ≤ 100 mol%, bezogen auf die Gesamtmenge der enthaltenen Oxyalkylengruppen.

Bevorzugt ist das NCO-terminierte Polyurethan-Prepolymer erhältlich aus der Reaktion einer Reaktionsmischung umfassend ein niedermolekulares, aliphatisches Diisocyanat und ein Polyoxyethylengruppen enthaltendes Polyol. Hinsichtlich der niedermolekularen, aliphatischen Diisocyanate sind Hexamethylendiisocyanat und Isophorondiisocyanat sowie deren Gemische bevorzugt. Hinsichtlich der Polyole sind Copolymere aus Ethylenoxid und Propylenoxid mit einem Ethylenoxidgehalt, bezogen auf die Gesamtmenge der enthaltenen Oxyalkylengruppen, von 60 bis 85 mol-% bevorzugt.

Bevorzugt umfasst das NCO-terminierte Polyurethan-Prepolymer weiterhin:
H1) ein oder mehrere niedermolekulare, aliphatische Diisocyanate mit einer Molmasse von ≥ 140 bis ≤ 278 g/mol, bevorzugt ≥ 168 bis ≤ 258 g/mol und/oder daraus herstellbare Polyisocyanate mit einer Isocyanatfunktionalität von ≥ 2 bis ≤ 6;
   und/oder
H2) ein oder mehrere monofunktionelle Polyalkylenoxide mit einer OH-Zahl von ≥ 10 bis ≤ 250 und einem Ethylenoxidanteil von ≥ 50 bis ≤ 100 mol%, bezogen auf die Gesamtmenge der enthaltenen Oxyalkylengruppen;
   und/oder
eine hydrophile Isocyanatkomponente, welche durch die Umsetzung von unter H1) genannten Komponenten mit unter H2) genannten Komponenten erhältlich ist.

Das NCO-terminierte Polyurethan-Prepolymer umfasst bevorzugt:
A) isocyanatfunktionelle Prepolymere mit einem Gewichtsanteil an niedermolekularen, aliphatischen Diisocyanaten mit einer Molmasse von ≥ 140 bis ≤ 278 g/mol, bevorzugt ≥ 168 bis ≤ 258 g/mol von ≤ 1.0 Gew.-% bezogen auf das Prepolymer, erhältlich durch Umsetzung von
A1) niedermolekularen, aliphatischen Diisocyanaten einer Molmasse von ≥ 140 bis ≤ 278 g/mol, bevorzugt > 168 bis ≤ 258 g/mol mit
A2) di- bis hexafunktionellen Polyalkylenoxiden einer OH-Zahl von ≥ 22.5 bis ≤ 112 mg KOH/g und einem Ethylenoxidanteil von ≥ 50 bis ≤ 100 mol%, bezogen auf die Gesamtmenge der enthaltenen Oxyalkylengruppen,
B) gegebenenfalls heterocyclische, 4-Ring oder 6-Ring-Oligomere von niedermolekularen, aliphatischen Diisocyanaten mit einer Molmasse von ≥ 140 bis ≤ 278 g/mol, bevorzugt ≥ 168 bis ≤ 258 g/mol,
D) gegebenenfalls Katalysatoren,
E) gegebenenfalls Alkalimetallsalze schwacher anorganischer Säuren,
F) gegebenenfalls Tenside,
G) gegebenenfalls ein- oder mehrwertige Alkohole,
H) die folgenden Komponenten:
   H1) ein oder mehrere niedermolekulare, aliphatische Diisocyanate einer Molmasse von ≥ 140 bis ≤ 278 g/mol, bevorzugt ≥168 bis ≤ 258 g/mol und/oder daraus herstellbare Polyisocyanate mit einer Isocyanatfunktionalität von ≥2 bis ≤ 6;
      und/oder
   H2) ein oder mehrere monofunktionelle Polyalkylenoxide mit einer OH-Zahl von ≥ 10 bis ≤ 250 und einem Ethylenoxidanteil von ≥ 50 bis ≤ 100 mol%, bezogen auf die Gesamtmenge der enthaltenen Oxyalkylengruppen;
      und/oder
   eine hydrophile Isocyanatkomponente, welche durch die Umsetzung von unter H1) genannten Komponenten mit unter H2) genannten Komponenten erhältlich ist.

Bevorzugt weisen die eingesetzten Prepolymere A) einen Restmonomergehalt (Monomere entsprechen niedermolekularen, aliphatischen Diisocyanaten) von unter 0.5 Gew.-% bezogen auf das Prepolymer auf. Dieser Gehalt kann durch entsprechend gewählte Einsatzmengen der Diisocyanate A1) und der Polyalkylenoxide A2) erreicht werden. Bevorzugt ist jedoch der Einsatz des Diisocyanats A1) im Überschuss und mit anschließender, bevorzugt destillativer, Abtrennung nicht umgesetzter Monomere.

Bei der Herstellung der isocyanatfunktionellen Prepolymere A) wird das Verhältnis der Polyalkylenoxide A2) zu den niedermolekularen, aliphatischen Diisocyanaten A1) typischerweise so eingestellt, dass auf 1 Mol OH-Gruppen des Polyalkylenoxide A2) 2 bis 20 Mol, bevorzugt mit 2 bis 10 Mol und besonders bevorzugt 5 bis 10 Mol NCO-Gruppen des niedermolekularen, aliphatischen Diisocyanats A1) kommen.

Die Herstellung der unter H) genannten hydrophilen Polyisocyanate erfolgt typischerweise durch Umsetzung von 1 Mol OH-Gruppen der monofunktionellen Polyalkylenoxidkomponente H2) mit 1.25 bis 15 Mol, bevorzugt mit 2 bis 10 Mol und besonders bevorzugt 2 bis 6 Mol NCO-Gruppen eines Polyisocyanates H1) mit einer Isocyanatfunktionalität von 2 bis 6, basierend auf aliphatischen Diisocyanaten. Beispielhaft für derartige Polyisocyanate H1) sind Biuret-Strukturen, Isocyanurate bzw. Uretdione basierend auf aliphatischen Diisocyanaten. Dabei werden das Polyisocyanat H1) und das Polyalkylenoxid H2) bevorzugt über eine Urethangruppe bzw. eine Harnstoffgruppe miteinander verknüpft, wobei besonders die Verknüpfung über Urethangruppen bevorzugt ist.

Der NCO-Gehalt der isocyanatfunktionellen Prepolymere A) beträgt bevorzugt 1.5 bis 4.5 Gew.-%, besonders bevorzugt 1.5 bis 3.5 Gew.-% und ganz besonders bevorzugt 1.5 bis 3.0 Gew.-%.

Beispiele für niedermolekulare, aliphatische Diisocyanate der Komponente A1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexyl)methan (HMDI) bevorzugt sind. Besonders bevorzugt sind BDI, HDI, IPDI, ganz besonders bevorzugt Hexamethylendiisocyanat und Isophorondiisocyanat.

Polyalkylenoxide der Komponente A2) sind bevorzugt Copolymere aus Ethylenoxid und Propylenoxid mit einem Ethylenoxidgehalt, bezogen auf die Gesamtmenge der enthaltenen Oxyalkylengruppen, von 50 bis 100 mol-%, bevorzugt von 60 bis 85 mol-%, gestartet auf Polyolen oder Aminen. Geeignete Starter dieser Art sind Glycerin, Trimethylolpropan (TMP), Sorbit, Pentaerythrit, Triethanolamin, Ammoniak oder Ethylendiamin.

Die Polyalkylenoxide der Komponente A2) besitzen typischerweise zahlenmittlere Molekulargewichte von 1000 bis 15000 g/mol, bevorzugt von 3000 bis 8500 g/mol.

Ferner besitzen die Polyalkylenoxide der Komponente A2) OH-Funktionalitäten von 2 bis 6, bevorzugt von 3 bis 6, besonders bevorzugt von 3 bis 4.

Gegebenenfalls einzusetzende Verbindungen der Komponente B) sind heterocyclische, 4-Ring oder 6-Ring-Oligomere von niedermolekularen, aliphatischen Diisocyanaten mit einer Molmasse von 140 bis 278 g/mol, bevorzugt ≥ 168 bis ≤ 258 g/mol, wie Isocyanurate, Iminooxadiazindione oder Uretdione der vorgenannten niedermolekularen, aliphatischen Diisocyanate.

Wurde mit überschüssigem niedermolekularen Diisocyanat gearbeitet, erfolgt anschließend die Entfernung des Überschusses an niedermolekularem, aliphatischen Diisocyanat bevorzugt durch Dünnschichtdestillation. Vor, während und nach der Reaktion oder der destillativen Abtrennung des Diisocyanatüberschusses können saure oder alkylierende Stabilisatoren, wie Benzoylchlorid, Isophthaloylchlorid, Methyltosylat, Chlorpropionsäure, HCl oder Antioxidantien, wie Di-tert-butylkresol oder Tocopherol zugesetzt werden. Der NCO-Gehalt der hydrophilen Polyisocyanate H) beträgt bevorzugt 0.3 bis 20 Gew.-%, besonders bevorzugt 2 bis 10 Gew.-% und ganz besonders bevorzugt 3 bis 6 Gew.-%.

Zur Beschleunigung der Harnstoff - bzw. Urethanbildung können in Komponente D) Katalysatoren eingesetzt werden. Dabei handelt es sich typischerweise um die dem Fachmann aus der Polyurethantechnologie bekannten Verbindungen. Bevorzugt sind hier Verbindungen der Gruppe bestehend aus katalytisch aktiven Metallsalzen, welche nicht unter Komponente E) fallen, Aminen, Amidinen und Guanidinen. Beispielhaft zu nennen sind Zinndibutyldilaurat (DBTL), Zinnacetat, 1 ,8-Diazabi-cyclo[5.4.0]undecen-7 (DBU), 1,5-Diazabicyclo[4.3.0]nonen-5 (DBN), 1 ,4-Diazabicy-clo[3.3.0]octen-4 (DBO), N-Ethylmorpholin (NEM), Triethylendiamin (DABCO), Pentamethylguanidin (PMG), Tetramethylguanidin (TMG), Cyclotetramethylguanidin (TMGC), n-Decyl-tetramethylguanidin (TMGD), n-Dodecyltetramethylguanidin (TMGDO), Dimethylaminoethyltetramethylguanidin (TMGN), 1,1,4,4,5,5-Hexamethylisobiguanidin (HMIB), Phenyltetramethylguanidin (TMGP) und Hexamethylenoctamethylbiguanidin (HOBG).

Bevorzugt ist der Einsatz von Aminen, Amidinen, Guanidinen oder deren Mischungen als Katalysatoren der Komponente D). Bevorzugt ist auch die Verwendung von 1,8-Diazabicyclo[5.4.0]undecen-7 (DBU).

Es kann aber auch auf Katalysatoren der Komponente D) verzichtet werden; dies ist bevorzugt.

Als Komponente E) werden Alkalimetallsalze schwacher anorganischer Säuren eingesetzt. Hierunter werden Alkalimetallsalze von anorganischen Säuren verstanden, deren korrespondierende freie Säuren in Wasser bei 25 °C einen pKs-Wert von > 4.0 aufweisen. Beispiele besonders geeigneter Alkalimetallsalze schwacher anorganischer Säuren sind Kaliumhydroxid, Natriumhydroxid, Kaliumcarbonat, Natriumcarbonat und Natriumhydrogencarbonat, wobei auch beliebige Mischungen dieser Salze mit eingeschlossen sind.

Zur Verbesserung der Schaumbildung, Schaumstabilität oder der Eigenschaften des resultierenden Polyurethan-Schaums können Verbindungen der Komponente F) eingesetzt werden, wobei solche Additive grundsätzlich alle an sich bekannten anionischen, kationischen, amphoteren und nichtionischen Tenside sowie Mischungen hieraus sein können. Bevorzugt werden Alkylpolyglycoside, EO/PO-Blockcopolymere, Alkyl- oder Arylalkoxylate, Siloxanalkoxylate, Ester der Sulfobernsteinsäure und/oder Alkali- oder Erdalkalimetallalkanoate eingesetzt. Besonders bevorzugt werden EO/PO-Blockcopolymere eingesetzt. Bevorzugt werden allein die EO/PO-Blockcopolymere als Komponente F) eingesetzt.

Zudem können zur Verbesserung bestimmter Schaumeigenschaften des resultierenden Polyurethan-Schaums Verbindungen der Komponente G) verwendet werden. Hierbei handelt es sich um grundsätzlich alle dem Fachmann an sich bekannten ein- und mehrwertigen Alkohole sowie Mischungen hieraus. Dies sind ein- oder mehrwertige Alkohole oder Polyole, wie Ethanol, Propanol, Butanol, Decanol, Tridecanol, Hexadecanol, Ethylenglykol, Neopentylglykol, Butandiol, Hexandiol, Decandiol, Trimethylolpropan, Glycerin, Pentaerythrit, monofunktionelle Polyetheralkohole und Polyesteralkohole, Polyetherdiole und Polyesterdiole.

Bei der Herstellung der unter H) aufgeführten hydrophilen Polyisocyanate, die, wie erwähnt optional zur Anwendung kommen, wird das Verhältnis der monofunktionellen Polyalkylenoxide H2) zu den niedermolekularen, aliphatischen Diisocyanaten H1) bevorzugt so eingestellt, dass auf 1 Mol OH-Gruppen der monofunktionellen Polyalkylenoxide 1.25 bis 15 Mol, bevorzugt 2 bis 10 Mol und besonders bevorzugt 2 bis 6 Mol NCO-Gruppen des niedermolekularen, aliphatischen Diisocyanats H1) kommen. Anschließend erfolgt die Allophanatisierung bzw. Biurethisierung und/oder Isocyanuratbildung bzw. Uretdionbildung. Werden die Polyalkylenoxide H2) über Urethangruppen an die aliphatischen Diisocyanate H1) gebunden, findet bevorzugt im Anschluss eine Allophanatisierung statt. Weiterhin ist bevorzugt, dass Isocyanurat-Struktureinheiten gebildet werden.

Die Umsetzung kann in Gegenwart von Urethanisierungskatalysatoren wie Zinnverbindungen, Zinkverbindungen, Aminen, Guanidinen oder Amidinen, oder in Gegenwart von Allophanatisierungskatalysatoren wie Zinkverbindungen erfolgen.

Die Umsetzung der Komponenten zur Herstellung der unter H) aufgeführten hydrophilen Polyisocyanaten erfolgt typischerweise bei 25 bis 140 °C, bevorzugt 60 bis 100 °C.

Es kann aber auch bei der Herstellung von H) auf Katalysatoren verzichtet werden; dies ist bevorzugt.

Beispiele für niedermolekulare, aliphatische Diisocyanate der Komponente H1) sind Hexamethylendiisocyanat (HDI), Isophorondiisocyanat (IPDI), Butylendiisocyanat (BDI), Bisisocyanatocyclohexylmethan (HMDI), 2,2,4-Trimethylhexamethylendiisocyanat, Bisisocyanatomethylcyclohexan, Bisisocyanatomethyltricyclodecan, Xylendiisocyanat, Tetramethylxylylendiisocyanat, Norbornandiisocyanat, Cyclohexandiisocyanat oder Diisocyanatododecan, wobei Hexamethylendiisocyanat (HDI), Isophorondiisocyanat (IPDI), Butylendiisocyanat (BDI) und Bis(isocyanatocyclohexy1)methan (HMDI) bevorzugt sind. Besonders bevorzugt sind BDI, HDI, IPDI, ganz besonders bevorzugt Hexamethylendiisocyanat und Isophorondiisocyanat. Beispiele für höhermolekulare Polyisocyanate H2) sind Polyisocyanate mit einer Isocyanatfunktionalität von 2 bis 6 mit Isocyanurat-, Urethan-, Allophanat-, Biuret-, Iminooxadiazintrion-, Oxadiazintrion- und/oder Uretdiongruppen auf Basis der im vorstehenden Abschnitt genannten aliphatischen und/oder cycloaliphatischen Diisocyanate.

Bevorzugt werden als Komponente H1) höhermolekulare Verbindungen mit Biuret-, Iminooxadiazindion-, Isocyanurat- und/oder Uretdiongruppen auf Basis von Hexamethylendiisocyanat, Isophorondiisocyanat und/oder 4,4'-Diisocyanatodicyclohexylmethan eingesetzt. Weiter bevorzugt sind Isocyanurate. Ganz besonders bevorzugt sind Strukturen auf Basis von Hexamethylendiisocyanat.

Die monofunktionellen Polyalkylenoxide H2) weisen eine OH-Zahl von 15 bis 250, bevorzugt von 28 bis 112, und einem Ethylenoxidanteil von 50 bis 100 mol%, bevorzugt von 60 bis 100 mol%, bezogen auf die Gesamtmenge der enthaltenen Oxyalkylengruppen auf.

Unter monofunktionellen Polyalkylenoxiden im Sinne der Erfindung sind Verbindungen zu verstehen, die nur eine isocyanatreaktive Gruppe, d.h. eine Gruppe, die mit einer NCO-Gruppe reagieren kann, aufweisen.

Die Herstellung von Polyalkylenoxiden H2) durch Alkoxylierung geeigneter Startermoleküle ist literaturbekannt (z.B. Ullmanns Enzyklopädie der technischen Chemie, 4. Auflage, Band 19, Verlag Chemie, Weinheim S. 31-38). Geeignete Startermoleküle sind insbesondere gesättigte Monoalkohole wie Methanol, Ethanol, n-Propanol, Isopropanol, n-Butanol, Isobutanol, sec-Butanol, Diethylenglykolmonobutylether sowie aromatische Alkohole wie Phenol oder Monoamine wie Diethylamin. Bevorzugte Startermoleküle sind gesättigte Monoalkohole der vorstehend genannten Art. Besonders bevorzugt werden Diethylenglykolmonobutylether oder n-Butanol als Startermoleküle verwendet. Die monofunktionellen Polyalkylenoxide H2) besitzen typischerweise zahlenmittlere Molekulargewichte von 220 bis 3700 g/mol, bevorzugt von 500 bis 2800 g/mol.

Die monofunktionellen Polyalkylenoxide H2) besitzen bevorzugt eine OH-Gruppe als isocyanatreaktive Gruppe.

Typischerweise werden die Komponenten A) bis H) in folgenden Mengen eingesetzt (zur Reaktion mit 0.1 bis 200 Gewichtsteilen Wasser in Schritt (II) des erfindungsgemäßen Verfahrens):
100 Gewichtsteile isocyanatfunktioneller Prepolymere A)
0 bis 30 Gewichtsteile heterocyclischer Oligomere B)
0 bis 1 Gewichtsteile an Katalysatoren D)
0 bis 5 Gewichtsteile Alkalimetallsalze schwacher anorganische Säuren E)
0 bis 10 Gewichtsteile Tenside F)
0 bis 20 Gewichtsteile Alkohole G)
0 bis 250 Gewichtsteile einer oder mehrerer unter H) genannten Komponenten.

Bevorzugt werden die Komponenten A) bis H) in folgenden Mengen eingesetzt (zur Reaktion mit 0.1 bis 100 Gewichtsteilen Wasser):
100 Gewichtsteile isocyanatfunktioneller Prepolymere A)
1 bis 30 Gewichtsteile heterocyclischer Oligomere B)
0 bis 1 Gewichtsteile an Katalysatoren D)
0.01 bis 5 Gewichtsteile Alkalimetallsalze schwacher anorganische Säuren E)
0 bis 5 Gewichtsteile Tenside F)
0 bis 10 Gewichtsteile Alkohole G)
10 bis 100 Gewichtsteilen einer oder mehrerer unter H) genannten Komponenten.

Besonders bevorzugt werden die Komponenten A) bis H) in folgenden Mengen eingesetzt (zur Reaktion mit 1 bis 60 Gewichtsteilen Wasser):
100 Gewichtsteile isocyanatfunktioneller Prepolymere A)
5 bis 15 Gewichtsteile heterocyclischer Oligomere B)
0 bis 0,5 Gewichtsteile an Katalysatoren D)
0.1 bis 1 Alkalimetallsalze schwacher anorganische Säuren E)
0 Gewichtsteile Tenside F)
0 Gewichtsteile Alkohole G)
20 bis 80 Gewichtsteilen einer oder mehrerer unter H) genannten Komponenten.

In einer bevorzugten Ausführungsform des erfindungsgemäßen Verfahren ist die Komponente A1) ausgewählt aus der Gruppe bestehend aus Hexamethylendiisocyanat, Trimethylhexamethylendiisocyanant, Isophorondiisocyanat, 4,4'-Methylenbis(cyclohexylisocyanat) oder einer Mischung aus mindestens zwei hiervon.

In einer bevorzugten Ausführungsform des erfindungsgemäßen Verfahren weist die Schicht mindestens eine der folgenden Eigenschaften auf:
i. Eine Wasserdampfdurchlässigkeit (MVTR) von ≥ 1500 g/m²/24h, bevorzugt von > 2500 g/m²/24h;
ii. Eine Retention (bestimmt mit Hilfe eines Gewichts von 6 kg über 20 Sekunden) von mindestens 25 %, bevorzugt mindestens 35 % (g/g), bezogen auf maximale Absorption gemäß DIN EN 13726-1:2002, Teil 3.2 / 3.3;
iii. Eine Dicke von mindestens 0,1 mm, bevorzugt in einem Bereich von 0,1 bis 50 mm, oder bevorzugt in einem Bereich von 1 bis 25 mm, oder bevorzugt in einem Bereich von 1,5 bis 15 mm;
iv. Eine Dichte in einem Bereich von 70 bis 200 g/l, bevorzugt in einem Bereich von 80 bis 170 g/l, oder bevorzugt in einem Bereich von 100 bis 150 g/l.

Im erfindungsgemäßen Verfahren beinhaltet die erste Folie und/oder die weitere Folie ein Material ausgewählt aus der Gruppe bestehend aus einem thermoplastischen Polyurethan, einem Polyurethan oder einer Mischung hieraus. Das Material der ersten und/oder der weiteren Folie und/oder einer optionalen dritten Folie unterscheiden sich bevorzugt von dem Material der Schicht durch mindestens eine Eigenschaft, bevorzugt ausgewählt aus der Gruppe bestehend aus einer höheren Dichte, einer geringeren Wasserdampfdurchlässigkeit, einer geringeren Bruchdehnung, einer geringeren Dicke oder einer Kombination aus mindestens zwei hiervon.

In einer bevorzugten Ausführungsform des erfindungsgemäßen Verfahren weist die erste Folie und/oder die weitere Folie mindestens eine der folgenden Eigenschaften auf:

| | |
|---|---|
| EF1 | EF1 Eine Dicke in einem Bereich von 15 bis 75 µm, oder bevorzugt in einem Bereich von 20 bis 50 µm, oder bevorzugt in einem Bereich von 25 bis 45 µm; |
| EF2 | Eine Wasserdampfdurchlässigkeit von ≥ 1000 g/d m², oder bevorzugt von ≥1500 g/d m², oder bevorzugt in einem Bereich von 1000 bis 20000 g/d m², |
| | oder bevorzugt in einem Bereich von 1500 bis 10000 g/d m² (bestimmt nach DIN 13726-2:2002, Teil 3.2); |
| EF3 | Eine Bruchdehnung von ≥ 200%, bevorzugt ≥ 400 %, oder bevorzugt in einem Bereich von 200 bis 4000 %, oder bevorzugt in einem Bereich von 500 bis 3000 % (bestimmt nach DIN EN ISO 527-2); |
| EF4 | Aussparungen beinhaltet, die bevorzugt mindestens 3%, oder bevorzugt mindestens 5 %, oder bevorzugt mindestens 10 % ausmachen, oder bevorzugt in einem Bereich von 3 bis 30 %, oder bevorzugt in einem Bereich von 5 bis 25 %, oder bevorzugt in einem Bereich 10 bis 20 % bezogen auf die Gesamtfläche der jeweiligen Folie liegen; |
| EF5 | Ist eine Bakterien- und Virusbarriere gemäß ASTM F1671 (amerikanische Norm), bevorzugt weisen die erste oder die weitere Folie dann keine Aussparungen auf. |

Bevorzugt weist die erste Folie und/oder die weitere Folie die Merkmale EF1 und EF2, oder EF2 und EF3, oder EF1 und EF4, oder EF1 und EF5, oder EF2 und EF3, oder EF2 und EF4, oder EF2 und EF5, oder EF3 und EF4, oder EF4 und EF5, oder EF3 und EF4, oder EF3 und EF5, oder EF4 und EF5, oder EF1 und EF2 und EF3, oder EF1 und EF2 und EF4, oder EF1 und EF2 und EF5, oder EF1 und EF3 und EF4, oder EF1 und EF3 und EF5, oder EF2 und EF3 und EF4, oder EF2 und EF4 und EF5, oder EF3 und EF4 und EF5, oder EF1 und EF2 und EF3 und EF4, oder EF1 und EF2 und EF3 und EF5, oder EF1 und EF2 und EF4 und EF5, oder EF1 und EF3 und EF4 und EF5, oder EF2 und EF3 und EF4 und EF5, oder EF1 und EF2 und EF3 und EF4 und EF5 auf. Weiterhin bevorzugt sind Folien die Merkmalskombinationen aufweisen, wobei Merkmal EF4 nicht zusammen mit Merkmal EF5 auftritt.

In einer bevorzugten Ausführungsform des erfindungsgemäßen Verfahrens weist mindestens die erste Folie und/oder die weitere Folie Aussparungen auf, die einen Durchmesser in einem Bereich von 1 bis 8 mm, bevorzugt von 1,5 bis 7 mm, oder bevorzugt von 2 bis 5 mm aufweisen. Die Aussparungen sind bevorzugt regelmäßig über der jeweiligen Folie verteilt. Bevorzugt weist die jeweilige Folie mit Aussparungen eine Anzahl von Aussparungen in einem Bereich von 1 bis 10 Aussparungen pro cm², oder bevorzugt in einem Bereich von 2 bis 8 Aussparungen pro cm², oder bevorzugt in einem Bereich von 3 bis 7 pro cm² auf. Die Aussparungen können jede Form aufweisen, die der Fachmann hierfür auswählen würde. Bevorzugt weisen die Aussparungen eine kreisförmige Form auf. Bevorzugt werden die Aussparungen durch Ausstanzen der entsprechenden Fläche aus der ersten Folie oder der weiteren Folie gebildet.

Bevorzugt beinhaltet die Schicht des in dem erfindungsgemäßen Verfahren hergestellten Verbundmaterial ein Polyurethan, das sich aus dem NCO-terminierten Polyurethan-Prepolymer zusammen mit dem Wasser bildet. Es können in Schritt (II) bevorzugt weitere NCO-terminierte Polyurethan-Prepolymere zeitgleich oder nacheinander hergestellt werden und in Schritt (III) zeitgleich oder nacheinander auf die erste Folie appliziert werden. Aus der mindestens einen Prepolymer-Wassermischung entsteht nach dem Applizieren in Schritt (III) die Schicht, die mindestens eine ein Polyurethan enthaltende Lage beinhaltet. Wenn mehr als eine Lage einer Polyurethan-Prepolymer-Wasser-Mischung auf die erste Folie appliziert wird, weisen die mindestens zwei Lagen entweder die gleichen oder unterschiedliche Polyurethane, also mindestens ein erstes Polyurethan und gegebenenfalls mindestens ein weiteres Polyurethan nach dem Aushärten der Mischung auf. Das mindestens eine weitere Polyurethan weist bevorzugt mindestens eine unterschiedliche Eigenschaft, bevorzugt ausgewählt aus der Gruppe bestehend aus Wasserdampfdurchlässigkeit, Dicke, Retention und Dichte im Vergleich zu dem ersten Polyurethan auf. Bevorzugt unterscheidet sich mindestens eine der zuvor genannten Eigenschaften des ersten Polyurethans von dem weiteren Polyurethan um mindestens 10 % des jeweiligen Wertes der Eigenschaft des ersten Polyurethans.

Erfindungsgemäß befindet sich nach dem Applizieren in Schritt (III) kein zusätzliches Material außer dem in Schritt (III) aufgebrachten Polyurethan zwischen der ersten Folie und der Schicht. Erfindungsgemäß befindet sich nach dem Applizieren in Schritt (III) kein weiteres Material außer dem in Schritt (III) aufgebrachten Polyurethan zwischen der Schicht und der weiteren Folie. Erfindungsgemäß befindet sich nach dem Applizieren in Schritt (III) kein weiteres Material außer dem in Schritt (III) aufgebrachten Polyurethan zwischen der Schicht und der ersten Folie sowie zwischen der Schicht und der weiteren Folie. Im Rahmen der Erfindung wird unter dem Merkmal, dass das Verbundmaterial kein zusätzliches Material zwischen der Schicht und den Folien aufweist verstanden, dass außer den Materialien, die zur Bildung der ersten Folie unter I., der Schicht unter II. sowie der weiteren Folie unter III. keinerlei weitere oder zusätzliche Materialien verwendet werden, die zwischen der ersten Folie und der Schicht sowie der Schicht und der weiteren Folie angeordnet sind und bevorzugt zur Verbindung der Folien mit der Schicht beitragen. Es können sich jedoch weitere Schichten im inneren des Verbundmaterials befinden, die kein Polyurethan aufweisen. Diese weiteren Schichten treten jedoch nicht mit der ersten oder der weiteren Folie in direkten Kontakt.

In einer bevorzugten Ausführungsform des erfindungsgemäßen Verfahrens weist die Wundauflage mindestens eine der folgenden Eigenschaften auf:

| | |
|---|---|
| WA1. | Eine Dicke in einem Bereich von 1 mm bis 10 cm, bevorzugt von 1,2 mm bis 2,5 cm, oder bevorzugt in einem Bereich von 1,4 bis 10 mm; |
| WA2. | Ein Quellverhalten bei der Absorption von Wasser von kleiner 200 Vol.-%, bevorzugt von kleiner 180 Vol.-%, bevorzugt von kleiner 150 Vol.-%; |
| WA3. | Eine Ausdehnung in jeweils eine der Raumrichtungen, die in der Ebene liegen, die der größten Flächenausdehnung der Wundauflage entspricht von < 50%, bevorzugt von < 35 %, oder bevorzugt von < 20 %, bezogen auf die Ausdehnung der Wundauflage in die dritte Raumrichtung, bei Kontaktieren mit einer Flüssigkeit; |
| WA4. | Einer Kombination aus mindestens zwei der Eigenschaften WA1. bis WA3, bevorzugt die Merkmale WA1 und WA2, oder WA1 und WA3, oder WA2 und WA3, oder WA1 und WA2 und WA3. |

### Methoden:

Sofern nicht abweichend gekennzeichnet, beziehen sich alle Prozentangaben auf das Gewicht und die Gesamtmenge bzw. auf das Gesamtgewicht der Zusammensetzungen.

Sofern nicht abweichend vermerkt, beziehen sich alle analytischen Messungen auf Messungen bei Temperaturen von 23 °C.

### Bestimmung der Bruchspannung;

Die Bruchspannung wurde bestimmt mittels DIN EN ISO 527-2.

### Dickenmessung:

Die Schichtdickenbestimmung wird mit einem Drucklufttaster und zur Ausgabe der Schichtdicke angeschlossenem Display der Firma DR. JOHANNES HEIDENHAIN GmbH, Germany (MT25P) ermittelt.

### Dichtemessung:

Zur Volumenbestimmung wird ein Probenstück mit Hilfe eines Stanzeisens in der Dimension 5 x 5 cm² (mit abgerundeten Ecken und Kurvenradius von 3 mm) ausgestanzt. Die Höhe/Dicke wird aus dem Mittelwert einer 5fach-Bestimmung mittels oben beschriebener Methode gemessen. Zur anschließenden Berechnung der Dichte wird die Masse des Probenstückes an einer Mettler Toledo XS603S Waage bestimmt.

### Retention:

Für die Bestimmung der Retention wird ein Schaumstück der Größe 5 x 5 cm² nach vollständiger Absorption mit einer Pinzette angehoben und für 30 Sekunden abtropfen gelassen und gewogen. Danach wird das Schaumstück auf ein Metallpodest gelegt und mit einem Gewicht von 6 kg für 20 Sekunden beschwert. Nach Entfernen des Gewichtes wird das Schaumstück erneut gewogen und die verbliebene Feuchtigkeitsmenge im Vergleich prozentual zur maximalen Absorption bestimmt.

### Bestimmung der MVTR (Moisture vapor transmission rate)

Die Bestimmung der MVTR erfolgt in Anlehnung an die DIN EN13726-2:2002 (Teil 3.2). Dabei wird ein Metallzylinder, wie in der DIN beschrieben mit Wasser gefüllt und durch den zu untersuchenden Film bzw. die zu untersuchende Schicht an der Oberseite verschlossen. Anschließend wird das Gesamtgewicht (Becher mit Wasser und Film) mittels Waage bestimmt. Der Messaufbau wird für 24 h bei 37°C gelagert und wiederum das Gewicht bestimmt. Durch Subtraktion lässt sich der Wasserverlust, der durch den Film verdampft, ermitteln. Die MVTR wird in g/(m²*24 h) oder g/ m²/24 h angegeben.

### Bestimmung des maximalen Absorptionsvermögen von Wasser:

Die Bestimmung des maximalen Absorptionsvermögens erfolgte gemäß DIN EN 13726-1:2002 Teil 3.2 an einem flachen Schaumstück der Größe 5 * 5 cm².

### Beispiele:

### Erfindungsgemäße Herstellung einer klebstofffreien Wundkontaktmaterials (Beispiel B 1)

125 g Baymedix FP505 wurden mit 25 g einer wässrigen Lösung aus 1,32 Gew.-% Natriumhydrogencarbonat, 4.8 Gew.-% Pluronic PE6800 und 0.4% Zitronensäure-Monohydrat unter heftigem Rühren für 7s mit Hilfe eines Rührwerks mit Ankerrührblatt (Labordissolver green 037 der Firma Pendraulik GmbH) bei 930 U/min. vermischt und danach auf eine PU-Folie des Typs VPT9101 (Covestro Deutschland AG) mittels einer Rakel von 1,5 mm Spalt in x-y Richtung aufgebracht. Danach wurde die Oberfläche der Reaktionsmischung, die sich in x-y Richtung erstreckt, mit einer weiteren PU-Folie des Typs VPT9101 abgedeckt. Die Deckfolie hat kreisrunde Löcher mit einem Durchmesser von 4 mm und jeweils einen Abstand von 5 mm zwischen den Lochmittelpunkten in reihenförmiger Anordnung, wobei die Reihen versetzt zueinander angeordnet waren. Spätestens nach einer Wartezeit von 30 Minuten war der so entstandene, erfindungsgemäße Verbund als Wundkontaktmaterial einsetzbar.

Die Schicht in Form des trockenen Schaums (8 mm dick) zeigte eine maximale Absorption von 1100 %, eine Quellung von 30 Vol.-% bei einer Ausdehnung von 3 mm in x-y-Richtung (ausgehend von einem 100 cm² Messstück in x-y Richtung).

Bei dem Versuch den Verbund wieder in seine ursprünglichen Bestandteile, nämlich die zwei PU-Folien des Typs VPT9101 und den Schaum zu separieren, musste aufgrund der hohen Klebkraft zwischen PU-Folien und dem Schaum eine so hohe Kraft aufgewendet werden, dass der Schaum einen Kohäsionsbruch erlitt und folglich Schaumreste auf den PU-Folien zurückblieben.

### Vergleichsbeispiel (VB 1)

### Herstellung wie im Vergleichsbeispiel 1 außer:

Die Applizierung des Reaktionsmischung erfolgte auf einem Trennpapier des Typs Y 05200 der Felix Schöller Group, Osnabrück und die Abdeckung erfolgte durch auf dem Trennpapier gleichen Typs jedoch in einer genadelten Variante:
Der trockene Schaum (5 mm dick) zeigte eine maximale Absorption von 1500 %, eine Quellung von 180 Vol.-% und 40 mm in x-y-Richtung (ausgehend von einem 100 cm² Messstück in x-y-Richtung).

Bei dem Versuch den Verbund wieder in seine ursprünglichen Bestandteile, nämlich die zwei Trennpapiere des Typs Y 05200 und den Schaum zu separieren, war die Klebkraft zwischen den Trennpapieren und dem Schaum so gering, dass ein Abtrennen der Trennpapier ohne Rückstände des Schaums leicht möglich war, was einem Adhäsionsbruch gleich kommt, ohne dass die Schicht zerstört wird oder Risse erleidet.

### Figuren

In der folgenden Figurenbeschreibung wird exemplarisch das Verfahren zur Herstellung des Verbundmaterials ausgeführt sowie die Anordnung der Folien und der Schicht in dem erfindungsgemäßen Verbundmaterial gezeigt. Dabei zeigt
- Figur 1:: eine schematische Darstellung des erfindungsgemäßen Verfahrens mit den Schritten (I) bis (IV);
- Figur 2:: eine schematische Darstellung eines Verbundmaterials;
- Figur 3:: eine schematische Darstellung einer Wundauflage.

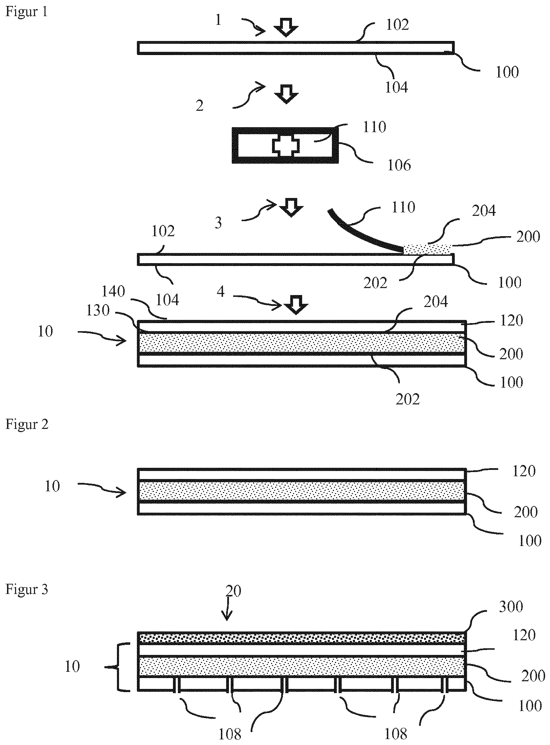

In Figur 1 wird in Schritt (I), entsprechend Schritt (I) des erfindungsgemäßen Verfahrens die erste Folie 100 bereitgestellt. Bevorzugt ist diese Folie 100 eine Polyurethan-Folie mit einer Dicke von 20 bis 50 µm. Die erste Folie 100 weist eine erste Folienoberfläche 102 sowie eine der ersten Folienoberfläche 102 gegenüberliegende zweite Folienoberfläche 104 auf. In Schritt (II), entsprechend Schritt (II) des erfindungsgemäßen Verfahrens wird ein NCO-terminiertes Polyurethan-Prepolymer mit Wasser unter Erhalt einer Polyurethan-Prepolymer-Wasser-Mischung 110 in einem Mischer 106 gemischt. Das Gewichtsverhältnis des NCO-terminierten Polyurethan-Prepolymers zu Wasser liegt dabei zwischen 100 : 10 und 100 : 50, bevorzugt 100 : 20. Die Mischung 110 wird in Schritt (III), entsprechend Schritt (III) des erfindungsgemäßen Verfahrens, mittels einer Stahl-Rakel (Film Applicator System Wasag Modell 288 der Firma ERICHSEN GmbH & Co.KG, Deutschland mit einer Rakelspalthöhe von 1,5 mm)auf die erste Folienoberfläche 102 der bereitgestellten Folie 100 in einer Nassfilmdicke von 1500 µm aufgetragen. Es bildet sich innerhalb weniger Sekunden bis weniger Minuten eine Schicht 200 aus der Mischung 110 auf der Folie 100 aus. Bevor die Schicht 200 komplett ausgeschäumt ist, wird in Schritt (IV), entsprechend Schritt (IV) des erfindungsgemäßen Verfahrens, eine weitere Folie 120 mit ihrer weiteren Folienoberfläche 130 auf die Schicht 200 aufgebracht. Bevorzugt erfolgt dies über ein Rolle zu Rolle Verfahren direkt nach Aufbringen der Mischung 110 auf die erste Folie 100, die ebenfalls in Form einer Rolle zu Rolle Anordnung bereitgestellt wird. Bevorzugt erfolgt die Auftragung der Folie 120 bereits unmittelbar, also so schnell wie technisch möglich, nach Aufbringen der Reaktionsmischung 200. In diesem Beispiel findet das Aufbringen der Folie 120 auf die in Schritt (III) aufgebrachte Mischung 110 innerhalb von wenigen Sekunden, bevorzugt in einem Zeitraum von 1 bis 90 Sekunden, oder bevorzugt in einem Zeitraum von 2 bis 60 Sekunden, statt. Der in Schritt (IV) entstehende Verbund 10 entspricht einem erfindungsgemäßen Verbundmaterial 10, das zu einer Wundauflage 20 weiterverarbeitet werden kann.

In Figur 2 ist ein Verbundmaterial 10 schematisch dargestellt. Das Verbundmaterial 10 ist aufgebaut aus einer ersten Folie 100, die über eine erste Folienoberfläche 102 mit einer Schicht 200 über deren erste Schichtoberfläche 202 verbunden ist. Auf der der ersten Schichtoberfläche 202 befindlichen zweiten Schichtoberfläche 204 der Schicht 200 ist die Schicht mit einer weiteren Folie 120 verbunden. Die erste Folie 100, die in diesem Fall dazu ausgelegt ist mit der Haut des Anwenders und damit mit der Wunde in Kontakt zu kommen, kann bevorzugt Aussparungen 108 (wie in Figur 3 gezeigt) in der ersten Folie 100, aber auch in der weiteren Folie 120 (hier nicht gezeigt) aufweisen. Bevorzugt weist jedoch lediglich diejenige Folie Aussparungen 108 auf, die dazu ausgelegt ist mit der Haut in Kontakt zu kommen. Die Rauigkeit der Folien 100 und 120 kann sich auf den nach innen (102, 130) bzw. nach außen weisenden Folienoberflächen (104, 140) stark unterscheiden. Weiterhin kann der Verbund 10 eine Bakterien- und/oder Virusbarriere aufweisen. Dies kann entweder dadurch erfolgen, dass mindestens eine der Folien 100 und/oder 120 als solch eine Barriere mit Eigenschaften gemäß ASTM F1671 ausgestaltet ist, oder der Schaum 200 Wirkstoffe beinhaltet, der eine solche Barriere ermöglichen.

Figur 3 zeigt schematisch eine Wundauflage 20, die das Verbundmaterial 10 enthält, das wie in Figur 2 beschrieben aufgebaut ist und wie in Figur 1 beschrieben hergestellt wurde. Neben dem Verbundmaterial 10 weist die Wundauflage 20 optional ein zusätzliches Material 300, bevorzugt in Form eine dritten Folie, auf der der Schicht 200 gegenüberliegenden Folienoberfläche 104 der weiteren Folie 120 auf. Dieses zusätzliche Material 300, bzw. die dritte Folie 300 dient vor allem dem Schutz des Verbundmaterials 10 gegenüber äußeren Einflüssen. Bevorzugt schützt die dritte Folie das Verbundmaterial 10 bei Benutzung der Wundauflage 20 vor einem Einreißen oder Abreißen. Optional oder alternativ kann die Dicke der weiteren Folie 120 auch so angepasst, also bevorzugt erhöht werden, dass ein schnelles Reißen bei hoher Beanspruchung vermieden wird. Jedoch kann aufgrund der Materialwahl des zusätzlichen Materials 300 auch eine unerwünschte Dicke des Verbundmaterials 10 vermieden werden. In die erste Folie 100, die in diesem Fall mit der Haut des Anwenders und damit mit der Wunde in Kontakt kommt, sind Aussparungen 108 in die Folie 100 eingearbeitet worden.

## Patentansprüche

1. Ein Verfahren zur Herstellung eines Verbundmaterials (10) für Wundauflagen (20), mit den Schritten:
(I) Bereitstellen einer ersten Folie (100), mit einer ersten Folienoberfläche (102) und eine zur ersten Folienoberfläche (102) nahezu parallel verlaufenden zweiten Folienoberfläche (104);
(II) Mischen eines NCO-terminierten Polyurethan-Prepolymers mit mindestens Wasser unter Erhalt einer Polyurethan-Prepolymer-Wasser-Mischung (110),
(III) Applizieren der Polyurethan-Prepolymer-Wasser-Mischung auf mindestens einen Teil der ersten Folienoberfläche (102) unter Bildung einer Schicht (200), wobei die Schicht (200) eine erste Schichtoberfläche (202) aufweist, die mit der ersten Folie (100) über mindestens einen Teil der ersten Folienoberfläche (102) in Kontakt steht und eine zweite Schichtoberfläche (204) aufweist, die zur ersten Schichtoberfläche (202) nahezu parallel verläuft;
(IV) Bedecken mindestens eines Teils der zweiten Schichtoberfläche (204) der noch flüssigen Schicht (200) mit mindestens einem Teil einer weiteren Folienoberfläche einer weiteren Folie (120) unter Erhalt des Verbundmaterials (10),
wobei die Klebkraft zwischen der ersten Folie (100) und der Schicht (200) sowie zwischen der Schicht (200) und der weiteren Folie (120) jeweils größer ist als die Bruchspannung der Schicht (200),
wobei das Verbundmaterial (10) zwischen der ersten Folie (100) und der Schicht (200) sowie zwischen der weiteren Folie (120) und der Schicht (200) kein weiteres Material aufweist und
wobei die erste Folie (100) und/oder die weitere Folie (120) ein Material ausgewählt aus der Gruppe bestehend aus einem thermoplastischen Polyurethan, einem Polyurethan oder einer Mischung hieraus beinhaltet.

2. Das Verfahren nach Anspruch 1, wobei in Schritt (IV) die weitere Folie (120) von Rolle zu Rolle auf die noch flüssige Schicht (200) abgelegt wird.

3. Das Verfahren nach Anspruch 1 oder 2, wobei mindestens die Schritte (III) und (IV), in einem Zeitraum von weniger als 120 Sekunden erfolgen.

4. Das Verfahren nach einem der vorhergehenden Ansprüche, wobei die Schicht (200) eine Dicke nach dem Aushärten aufweist, die um das 1,5 bis 30-fache größer ist als die Dicke beim Applizieren der Polyurethan-Prepolymer-Wasser-Mischung in Schritt (III).

5. Das Verfahren nach einem der vorhergehenden Ansprüche, wobei das NCO-terminierte Polyurethan-Prepolymer einen Gewichtsanteil an niedermolekularen, aliphatischen Diisocyanaten mit einer Molmasse von ≥ 140 bis ≤ 278 g/mol von ≤ 1.0 Gew.-%, bezogen auf das Prepolymer, aufweist und als Komponente A) erhältlich ist durch Umsetzung von
A1) niedermolekularen, aliphatischen Diisocyanaten einer Molmasse von ≥ 140 bis ≤ 278 g/mol mit
A2) di- bis hexafunktionellen Polyalkylenoxiden einer OH-Zahl von ≥ 22.5 bis ≤ 112 mg KOH/g und einem Ethylenoxidanteil von ≥ 50 bis ≤ 100 mol%, bezogen auf die Gesamtmenge der enthaltenen Oxyalkylengruppen.

6. Das Verfahren nach dem vorhergehenden Anspruch, wobei die Komponente A1) ausgewählt ist aus der Gruppe bestehend aus Hexamethylendiisocyanat, Trimethylhexamethylendiisocyanant, Isophorondiisocyanat, 4,4'-Methylenbis(cyclohexylisocyanat) oder einer Mischung aus mindestens zwei hiervon.

7. Das Verfahren nach einem der vorhergehenden Ansprüche, wobei die Schicht (200) mindestens eine der folgenden Eigenschaften aufweist:
i. Eine Wasserdampfdurchlässigkeit (MVTR) von ≥ 1500 g/m²/24h,
ii. Eine Retention (bestimmt mit Hilfe eines Gewichts von 6 kg über 20 Sekunden) von mindestens 25 % (g/g), bezogen auf maximale Absorption,
iii. Eine Dicke von mindestens 0,1 mm,
iv. Eine Dichte in einem Bereich von 70 bis 200 g/1.

8. Das Verfahren nach einem der vorhergehenden Ansprüche, wobei die erste Folie (100) und/oder die weitere Folie (120) mindestens eine der folgenden Eigenschaften aufweist:,
| | |
|---|---|
| EF1 | Eine Dicke in einem Bereich von 15 bis 75 µm; |
| EF2 | Eine Wasserdampfdurchlässigkeit von ≥ 1000 g/m²/24h,; |
| EF3 | Eine Bruchdehnung von ≥ 200%, bevorzugt ≥ 500 % Bruchdehnung; |
| EF4 | Aussparungen (108) beinhaltet, die bevorzugt mindestens 3%, bezogen auf die Gesamtfläche der jeweiligen Folie (100, 120) ausmachen; |
| EF5 | Eine Bakterien- und Virusbarriere gemäß ASTM F1671 aufweisen. |

9. Das Verfahren nach einem der vorhergehenden Ansprüche, wobei mindestens die erste Folie (100) und/oder die weitere Folie (120) Aussparungen (108) aufweist, die einen Durchmesser in einem Bereich von 2 bis 10 mm aufweisen.

10. Das Verfahren nach einem der vorhergehenden Ansprüche, wobei die Wundauflage mindestens eine der folgenden Eigenschaften aufweist:
| | |
|---|---|
| WA1. | Eine Dicke in einem Bereich von 1 mm bis 10 mm; |
| WA2. | Eine Quellung bei Inkontaktbringen mit Wasser von kleiner 200 Vol.-%, bezogen auf das Volumen vor Inkontaktbringen mit Wasser; |
| WA3. | Eine Ausdehnung in jeweils eine der Raumrichtungen, die in der Ebene liegt, die der größten Flächenausdehnung der Wundauflage entspricht von < 50%, bezogen auf die Ausdehnung der Wundauflage in die dritte Raumrichtung, bei Kontaktieren mit einer Flüssigkeit, |
| WA4. | Einer Kombination aus mindestens zwei der Eigenschaften WA1. bis WA3. |

## Claims

1. Method for producing a composite material (10) for wound dressings (20), comprising the following steps:
(I) providing a first film (100), having a first film surface (102) and a second film surface (104) running virtually parallel to the first film surface (102);
(II) mixing an NCO-terminated polyurethane prepolymer with at least water to give a polyurethane prepolymer/water mixture (110),
(III) applying the polyurethane prepolymer/water mixture to at least one part of the first film surface (102) to form a layer (200), the layer (200) having a first layer surface (202) which is in contact with the first film (100) via at least one part of the first film surface (102), and having a second layer surface (204) which runs virtually parallel to the first layer surface (202) ;
(IV) covering at least one part of the second layer surface (204) of the still-liquid layer (200) with at least one part of a further film surface of a further film (120) to give the composite material (10),
wherein the bond strength between the first film (100) and the layer (200) and also between the layer (200) and the further film (120) is greater in each case than the tensile strain at break of the layer (200),
wherein the composite material (10) has no further material between the first film (100) and the layer (200) and also between the further film (120) and the layer (200), and
wherein the first film (100) and/or the further film (120) comprises a material selected from the group consisting of a thermoplastic polyurethane, a polyurethane or a mixture thereof.

2. Method according to Claim 1, wherein the further film (120) is preferably deposited from roll to roll onto the still-liquid layer (200) in step (IV).

3. Method according to Claim 1 or 2, wherein at least steps (III) and (IV) take place within a period of less than 120 seconds.

4. Method according to any of the preceding claims, wherein the layer (200) has a thickness after curing which is greater by 1.5 to 30 times than the thickness on application of the polyurethane prepolymer/water mixture in step (III).

5. Method according to any of the preceding claims, wherein the NCO-terminated polyurethane prepolymer has a weight fraction of low molecular mass, aliphatic diisocyanates having a molar mass of ≥ 140 to ≤ 278 g/mol of ≤ 1.0 wt%, based on the prepolymer, and is obtainable as component A) by reaction of
A1) low molecular mass, aliphatic diisocyanates with a molar mass of ≥ 140 to ≤ 278 g/mol with
A2) di- to hexa-functional polyalkylene oxides with an OH number of ≥ 22.5 to ≤ 112 mg KOH/g and an ethylene oxide fraction of ≥ 50 to ≤ 100 mol%, based on the total amount of the oxyalkylene groups present.

6. Method according to the preceding claim, wherein component A1) is selected from the group consisting of hexamethylene diisocyanate, trimethylhexamethylene diisocyanate, isophorone diisocyanate, 4,4'-methylenebis(cyclohexyl isocyanate) or a mixture of at least two thereof.

7. Method according to any of the preceding claims, wherein the layer (200) has at least one of the following properties:
i. a water vapour transmissibility (MVTR) of ≥ 1500 g/m²/24h,
ii. a retention (determined using a 6 kg weight over 20 seconds) of at least 25% (g/g), based on maximum absorption,
iii. a thickness of at least 0.1 mm,
iv. a density in a range from 70 to 200 g/l.

8. Method according to any of the preceding claims, wherein the first film (100) and/or the further film (120) have/has at least one of the following properties:
EF1 a thickness in a range from 15 to 75 µm;
EF2 a water vapour transmissibility of ≥ 1000 g/m²/24h;
EF3 an elongation at break of ≥ 200%, preferably ≥ 500% elongation at break;
EF4 contains apertures (108) which make up preferably at least 3%, based on the total area of the respective film (100, 120);
EF5 exhibit a bacterial barrier and viral barrier in accordance with ASTM F1671.

9. Method according to any of the preceding claims, wherein at least the first film (100) and/or the further film (120) have/has apertures (108) which have a diameter in a range from 2 to 10 mm.

10. Method according to any of the preceding claims, wherein the wound dressing has at least one of the following properties:
WA1. a thickness in a range from 1 mm to 10 mm;
WA2. a swelling on contacting with water of less than 200 vol%, based on the volume before contacting with water;
WA3. an extent in one of the spatial directions that lies within the plane corresponding to the largest areal extent of the wound dressing of < 50%, based on the extent of the wound dressing in the third spatial direction, on contacting with a fluid;
WA4. a combination of at least two of the properties WA1. to WA3.

## Revendications

1. Procédé pour la fabrication d'un matériau composite (10) pour pansements (20), comportant les étapes :
(I) mise à disposition d'un premier film (100) ayant une première surface (102) de film et une deuxième surface (104) de film s'étendant sensiblement parallèlement à la première surface (102) de film ;
(II) mélangeage d'un prépolymère polyuréthane à terminaison NCO avec au moins de l'eau, avec obtention d'un mélange (110) de prépolymère polyuréthane et d'eau,
(III) application du mélange de prépolymère polyuréthane et d'eau sur au moins une partie de la première surface (102) de film avec pour formation d'une couche (200), la couche (200) présentant une première surface (202) de couche qui est en contact avec le premier film (100) sur au moins une partie de la première surface (102) de film et une seconde surface (204) de couche qui s'étend sensiblement parallèlement à la première surface (202) de couche ;
(IV) recouvrement d'au moins une partie de la seconde surface (204) de couche de la couche (200) encore fluide avec au moins une partie d'une surface supplémentaire de film d'un film supplémentaire (120) avec obtention du matériau composite (10),
dans lequel la force d'adhérence entre le premier film (100) et la couche (200) ainsi qu'entre la couche (200) et le film supplémentaire (120) est chaque fois supérieure à la contrainte de rupture de la couche (200), dans lequel le matériau composite (10) ne comporte pas d'autre matériau entre la couche (100) et la couche (200) ainsi qu'entre le film supplémentaire (120) et la couche (200) et
dans lequel le premier film (100) et/ou le film supplémentaire (120) comprend/comprennent une substance choisie dans le groupe constitué par un polyuréthane thermoplastique, un polyuréthane ou un mélange de ceux-ci .

2. Procédé selon la revendication 1, dans lequel, dans l'étape (IV), le film supplémentaire (120) est déposé de rouleau à rouleau sur la couche (200) encore fluide.

3. Procédé selon la revendication 1 ou 2, dans lequel au moins les étapes (III) et (IV) s'effectuent en un espace de temps de moins de 120 secondes.

4. Procédé selon l'une quelconque des revendications précédentes, dans lequel la couche (200) présente après le durcissement une épaisseur qui est 1,5 à 30 fois supérieure à l'épaisseur lors de l'application du mélange de prépolymère et d'eau dans l'étape (III).

5. Procédé selon l'une quelconque des revendications précédentes, dans lequel le prépolymère à terminaison NCO présente une proportion en poids de diisocyanates aliphatiques de faible masse moléculaire, ayant une masse molaire de ≥ 140 à ≤ 278 g/mole, de ≤ 1,0 % en poids, par rapport au prépolymère, et peut être obtenu en tant que composant A) par mise en réaction de
A1) diisocyanates aliphatiques de faible masse moléculaire, ayant une masse molaire de ≥ 140 à ≤ 278 g/mole, avec
A2) des polyoxyalkylènes di- à hexafonctionnels ayant un indice d'OH de ≥ 22,5 à ≤ 112 mg de KOH/g et une proportion d'oxyde d'éthylène de ≥ 50 à ≤ 100 % en moles, par rapport à la quantité totale des groupes oxyalkylène contenus.

6. Procédé selon la revendication précédente, dans lequel le composant A1) est choisi dans le groupe constitué par le diisocyanate d'hexaméthylène, le diisocyanate de triméthylhexaméthylène, le diisocyanate d'isophorone, le 4,4'-méthylènebis(cyclohexylisocyanate) ou un mélange d'au moins deux de ceux-ci.

7. Procédé selon l'une quelconque des revendications précédentes, dans lequel la couche (200) présente au moins les propriétés suivantes :
i. une perméabilité à la vapeur d'eau (MVTR) de ≥ 1 500 g/m²/24 h,
ii. une rétention (déterminée à l'aide d'un poids de 6 kg pendant 20 secondes) d'au moins 25 % (g/g), par rapport à l'absorption maximale,
iii. une épaisseur d'au moins 0,1 mm,
iv. une densité dans une plage de 70 à 200 g/l.

8. Procédé selon l'une quelconque des revendications précédentes, dans lequel le premier film (100) et/ou le film supplémentaire (120) présente(nt) au moins l'une des propriétés suivantes :
EF1 une épaisseur dans une plage de 15 à 75 µm ;
EF2 une perméabilité à la vapeur d'eau de ≥ 1000 g/m² /24 h ;
EF3 un allongement à la rupture de ≥ 200 %, de préférence un allongement à la rupture de ≥ 500 % ;
EF4 comporte(nt) des ouvertures (108) qui représentent de préférence au moins 3 %, par rapport à la surface totale du film (100, 120) respectif ;
EF5 comporte(nt) une barrière aux bactéries et virus selon ASTM F1671.

9. Procédé selon l'une quelconque des revendications précédentes, dans lequel au moins le premier film (100) et/ou le film supplémentaire (120) comporte(nt) des ouvertures (108) qui présentent un diamètre dans une plage de 2 à 10 mm.

10. Procédé selon l'une quelconque des revendications précédentes, dans lequel le pansement présente au moins une des propriétés suivantes :
WA1. une épaisseur dans une plage de 1 à 10 mm ;
WA2. un gonflement, à la mise en contact avec de l'eau, de moins de 200 % en volume, pat rapport au volume avant la mise en contact avec de l'eau ;
WA3. un expansion, dans chaque fois l'une des directions spatiales qui se situe dans le plan qui correspond à la plus grande extension superficielle du pansement, de < 50 %, par rapport à l'expansion du pansement dans la troisième direction spatiale, lors de la mise en contact avec un liquide,
WA4. une combinaison d'au moins deux des propriétés WA1. à WA3.
